# EUROPEAN PATENT SPECIFICATION

(11) **EP 1 539 048 B1**
(45) Date of publication and mention of the grant of the patent: **02.12.2009**
(21) Application number: 03733776.3
(22) Date of filing: 19.06.2003
(51) Int. Cl.: A61F 2/26, A61L 27/50, A61F 5/41

(54) **MULTI-MATERIAL PENIS CONSTRICTION DEVICE**
PENIS-KONSTRIKTIONSVORRICHTUNG AUS MEHREREN MATERIALIEN
DISPOSITIF DE CONSTRICTION DU PENIS CONSTITUE DE PLUSIEURS MATERIAUX

(30) Priority: 29.07.2002 US 398809 P
(43) Date of publication of application: 15.06.2005
(73) Proprietor: Potentica AG, 6340 Baar (CH)
(72) Inventor: FORSELL, Peter, CH-6300 Zug (CH)
(74) Representative: Strandin, Heléne
(86) International application number: PCT/SE2003/001056
(87) International publication number: WO 2004/010906

(56) References cited:
- WO-A2-01/47434
- US-A- 4 428 365
- US-A- 4 756 949
- US-A- 4 982 731
- US-A- 5 873 904

## Description

The present invention relates to an implantable constriction device for constricting penile blood vessels of a patient, for treating impotence.

This kind of constriction device in the form of a banding device, in which a band encircles a portion of a patient's penile exit veins or corpus cavernosa to restrict the patient's penile venous blood flow has been used in surgery to treat impotence. In practice, the band is made of silicone, which is a material approved and widely used for implantation. Moreover, the silicone band has an acceptable tensile strength and is fairly resistant to aggressive body fluids. Where the band is hydraulically adjusted the hydraulic fluid used typically is an isotonic salt solution mixed with other conventional materials.

A problem of traditional silicone bands, however, is that the silicone material gives the band certain inadequate properties, such as poor fatigue resistance and poor endurance of static bending forces, which over time might result in breakage of the band. Furthermore, silicone is a material that is semi-permeable by liquid, which is a drawback to hydraulic silicone bands, because hydraulic fluid can escape by diffusing through the silicone material. As a result, accurate adjustments of a hydraulic band are difficult to perform because of loss of hydraulic fluid and the patient has to regularly visit a doctor for adding hydraulic fluid to and calibrating the constriction device. These inadequate properties- are serious considering that the band should be implanted for the rest of the patient's life. Another problem is that the band somewhat restrains the dynamics movements of adjacent organs necessary for transportation of urine or fecal. As a consequence, the band might erode and over time injure the blood vessels or the corpus cavernosa.

The object of the present invention is to provide a new implantable constriction device for treating impotence having improved properties as compared to traditional constriction devices.

Accordingly, the present invention provides an implantable constriction device for constricting penile blood vessels of a patient, for treating impotence according to claim 1.

In accordance with a first embodiment of the invention, the property improving means comprises a coating coated on the base material at least along a side of the elongate composite structure that is intended to contact the penile veins or corpus cavernosa, wherein the coating has better aggressive body fluid resistance than the base material. Such a coating may comprise a Teflon™ or Parylene™ coating, or a biocompatible metal coating such as gold, silver or titanium. As a result, the constriction device can be protected from damaging influences of aggressive body fluids possibly for the rest of the patient's life.

Where the traditional silicone material constitutes the base material a Teflon™ or Parylene™ coating also provides the composite structure with better anti-friction properties than the base material. Good anti-friction properties of the composite structure reduce the risk of the elongate composite structure eroding the penile veins or corpus cavernosa. This is proven by tests, in which the surface of traditional silicone bands has been polished before use. Accordingly, the test results indicate significant improvements in avoiding erosion of the penile veins or corpus cavernosa.

Furthermore, the Teflon™, Parylene™ or metal coating also makes the composite structure, in which the base material is made of silicone, stronger than the traditional silicone band. A stronger band reduces the risk of fracture.

In one alternative of the first embodiment, the elongate composite structure is designed for mechanical adjustment, such as the mechanical solutions disclosed in WO 01/47434. In this alternative, the property improving means comprises a core of a soft viscoelastic material, such as silicone gel, typically having a hardness less than 20 Shore, cellulose gel or collagen gel. Where silicone gel is chosen it may be "Med 3 - 6300" manufactured by Nusil. Hard silicone constitutes the base material, typically having a hardness of at least 60 Shore, and covers the soft core of viscoelastic material. The soft core makes the implanted elongate composite structure less injurious to the penile veins or corpus cavernosa and reduces the injury of such organs. Furthermore, the soft core of viscoelastic material may be formed to enclose and protect mechanical adjustment components and other components of the composite structure, whereby fibrosis is prevented from growing into such components.

In another alternative of the first embodiment, the elongate composite structure is designed for hydraulic adjustment, such as the hydraulic solutions disclosed in WO 01/54626. In this alternative, the base material forms a closed tubing, which can be inflated by adding hydraulic fluid to the interior of the tubing and be deflated by withdrawing hydraulic fluid from the interior of the tubing. The coating of Teflon™, Parylene™ or metal may cover the inner surface of the tubing. The base material may form two coaxial tubular layers of hard silicon and the property improving means may comprise a tubular intermediate layer of a soft viscoelastic material located between the coaxial tubular layers. Alternatively, the base material may form an outer tubular layer, an inner arcuate layer attached to the outer tubular layer, the outer and inner layers defining a curved space extending longitudinally along the tubing. The property improving means may comprise a viscoelastic material filling the space. The tubing is applied around the penile veins or corpus cavernosa so that the space with viscoelastic material is located closest to the penile exit veins or corpus cavernosa. The viscoelastic material gives the advantages that erosion of the penile veins or corpus cavernosa is reduced and the risk of hydraulic fluid leaking from the tubing is decreased.

In a traditional silicone band, especially the tubular type, that is formed in a loop to constrict the penile veins or corpus cavernosa, the inner surface of the band loop that contacts the penile veins or corpus cavernosa forms bulges and creases that repeatedly changes as the band is subjected to dynamic movements from the penile veins or corpus cavernosa and when the size of the band is adjusted. As a consequence, the implanted traditional silicone band has the drawback that it may crack after some time due to fatigue of the silicone material With the elongate composite structure of the invention, in which polyurethane and silicone may constitute the base material and a fatigue resistant polyurethane layer covers the silicone material on the side of the elongate composite structure that contacts the penile veins or corpus cavernosa, this drawback is eliminated.

The property improving means suitably comprises a coating that is coated on the base material wherein the coating has better aggressive body fluid resistance properties and/or better anti-friction properties than hard silicone. As described above in connection with the first embodiment the coating may comprise a Teflon™ or Parylene™ coating, or a biocompatible metal coating.

The base material may form an inflatable tubing and the coating of polyurethane may cover the base material layer within the tubing.

In accordance with an embodiment of the invention, the base material forms an inflatable tubing and the property improving means comprises a liquid impermeable coating coated on the base material. The coating may be coated on the external and/or internal surface of the tubing. Preferably, the liquid impermeable coating comprises a Parylene™ coating, or a biocompatible metal coating. Where hard silicone, which is a liquid semi-permeable material, constitutes part of the base material, the coating of Parylene^{™} or metal gives the advantage that the tubing may be inflated by hydraulic fluid under pressure without risking fluid diffusing through the silicone wall of the tubing.

The base material may form two coaxial tubular layers of hard silicon and the property improving means may comprise a tubular intermediate layer of a soft viscoelastic material located between the coaxial tubular layers. Alternatively, the base material may form an outer tubular layer of hard silicone and an inner arcuate layer of silicone attached to the outer tubular layer. The outer and inner layers define a curved space extending longitudinally along the tubing and filled with the viscoelastic material. The tubing is intended to be applied around the penile veins or corpus cavernosa so that the space with viscoelastic material is located closest to the penile exit veins or corpus cavernosa.

In accordance with a further embodiment the property improving means comprises gas, such as air, contained in a multiplicity of cavities formed in the base material to improve the flexibility of the composite structure. In this case, Teflon™ advantageously constitutes the base material. The cavities may be defined by net structures of the Teflon™ material. Thus, the resulted composite structure of Teflon™ and cavities with gas will be strong, flexible and aggressive body fluid resistant, and have good tensile strength and good anti-friction properties.

Also in the fourth embodiment the elongate composite structure may comprise an inflatable tubing.

The present invention also provides an implantable constriction device for constricting penile blood vessels of a patient, for treating impotence, the constriction device comprising an elongate composite structure adapted to constrict the exit penile veins or corpus cavernosa of the patient to restrict the penile venous blood flow, wherein the composite structure includes an elongate biocompatible self-supporting base material having surfaces exposed to aggressive body cells when the constriction device is implanted in the patient, and a cell barrier coating coated on the surfaces to prevent body cells from breaking down the base material, typically of silicone. If the base material were broken down by such body cells, typically macrophages or killer cells, histological particles would be spread in the human body.

The barrier coating may comprise a Parylene™ coating or a biocompatible metal coating.

Alternatively, the barrier coating may comprise a composite of different materials to achieve the cell-barrier protection as described above. There are several examples of such composite materials on the market, for example a composite of polyurethane and silicone called Elaston™.

The invention is explaied in more detail in the following with reference to the accompanying drawings, in which:
Figure 1 is a front view of a mechanical constriction device,
Figure 2 is an enlarged cross-section along the line II-II in Figure 1,
Figures 3 and 4 are modifications of the embodiment shown in Figure 2,
Figure 5 is a front view of a hydraulic constriction device,
Figure 6 is an enlarged cross-section along the line VI-VI in Figure 5,
Figures 7 -10 are modifications of the embodiment shown in Figure 6, and
Figure 11 is a modification of the embodiment shown in Figure 2.

Referring to the drawing figures, like reference numerals designate identical or corresponding elements throughout the several figures.

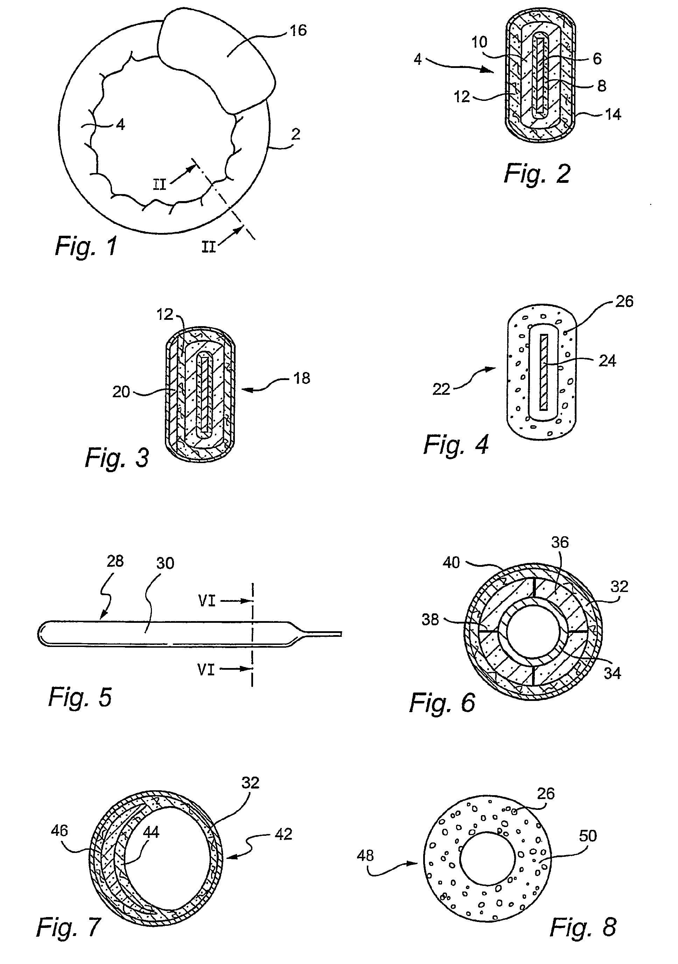

Figure 1 illustrates a mechanical constriction device 2 according to the invention comprising an elongate composite structure 4 adapted to extend around and constrict the exit penile veins or corpus cavernosa of a patient to restrict the penile venous blood flow therein. Referring to Figure 2, the elongate composite structure 4 comprises a strong band 6 of nylon or the like, a tubular layer 8 of hard silicone, in which the band 6 slides, a soft layer 10 of a viscoelastic-material, here a silicone gel having a hardness not more than 20 Shure, encircling the hard silicone layer 8, and a tubular layer 12 of a self-supporting base material of hard silicone having a hardness of at least 60 Shore, surrounding the soft silicon layer 10. A coating 14 of Teflon™, Parylene™ or a biocompatible metal, such as gold, silver or titanium, is coated on the outer hard silicone layer 12 to make the composite structure resistant to aggressive body fluids and to give the composite structure good anti-friction properties. A coating of Teflon^{™}, Parylene^{™} or metal may also be coated on the internal surface of the inner tubular hard silicone layer 8 to reduce the friction between the nylon band 6 and the layer 8. The constriction device 2 has an adjustment means 16 that can displace the end portions of the nylon band 6 relative to each other to either increase or decrease the constriction of the penile veins or corpus cavernosa.

Figure 3 shows an elongate composite structure 18 similar to that of Figure 2, except that a layer 20 of a fatigue resistant material, here polyurethane, is applied on the hard silicone layer 12 along the inner side of the structure 18 that is intended to contact the exit penile veins or corpus cavernosa. Alternatively, the layer 20 may be tubular and surround the layer 12.

Figure 4 shows a cross-section of an elongate composite structure 22 of an embodiment, in which Teflon^{™} constitutes the self-supporting base material, which is formed with a longitudinal cavity' in which a strong nylon band 24 slides. Property improving means in the form of gas, here air, contained in a multiplicity of cavities 26 are formed in the base material to improve the flexibility thereof.

Figure 5 shows a hydraulic constriction device 28 comprising an elongate composite structure in the form of an inflatable tubing 30, in which the base material of hard silicone forms an outer tubular layer 32 and an inner coaxial layer 34. A viscoelastic material, here soft silicone gel, forms an intermediate layer 36 located between the tubular layers 32,34. Four longitudinal partition walls 38 between the tubular layers 32,34 divide the intermediate layer 36 into four sections to prevent the silicone gel from displacing in the circumferential direction of the tubing 30. (Also the embodiments according to Figures 2 and 3 may be provided with such longitudinal partition walls.) The outer layer 32 is coated with a coating 40 of Teflon^{™}, Parylene^{™} or metal. Also the inner layer 34 may be coated with a coating of Teflon™, Parylene™ or metal. If a Parylene^{™} or metal coating is chosen the composite structure will be completely liquid impermeable.

Figure 7 shows a tubing 42 similar to that of Figure 6, except that an inner arcuate layer 44 is substituted for the inner tubular layer 34. The arcuate layer 44 is attached to the outer tubular layer 32, so that the outer tubular layer 32 and the arcuate layer 44 define a curved space extending longitudinally along the tubing 42. A viscoelastic material, here silicone gel 46, fills the space. In this embodiment there is no need for partition walls of the kind shown in the embodiment according to Figure 6. The tubing 42 is intended to be applied around the exit penile veins or corpus cavernosa so that the space with the protecting soft silicone gel 46 is located close to the exit penile veins or corpus cavernosa.

As taught by the embodiment of Figure 7, in the composite structures shown in Figures 2 and 3 the soft silicone gel may alternatively be applied in a longitudinal space close to the inner side of the elongate composite structure 4 and 18, respectively, that is intended to contact the exit penile veins or corpus cavernosa.

In the same manner as described above in connection with the embodiment of Figure 3, a layer of a fatigue resistant material, here polyurethane, may be applied on the outer tubular layer 32 of hard silicone of the tubing 30 and 42, respectively, along the side of the tubing 30 and 42, respectively, that is intended to contact the exit penile veins or corpus cavernosa, when the tubing 30 and 42, respectively, encircles the exit penile veins or corpus cavernosa.

Figure 8 shows a cross-section of an elongate composite structure 48 of an embodiment of the invention, in which Teflon^{™} constitutes the self-supporting base material, which is formed to an inflatable tubing 50. Property improving means in the form of gas contained in a multiplicity of cavities 26 are formed in the base material to improve the flexibility of the tubing 50.

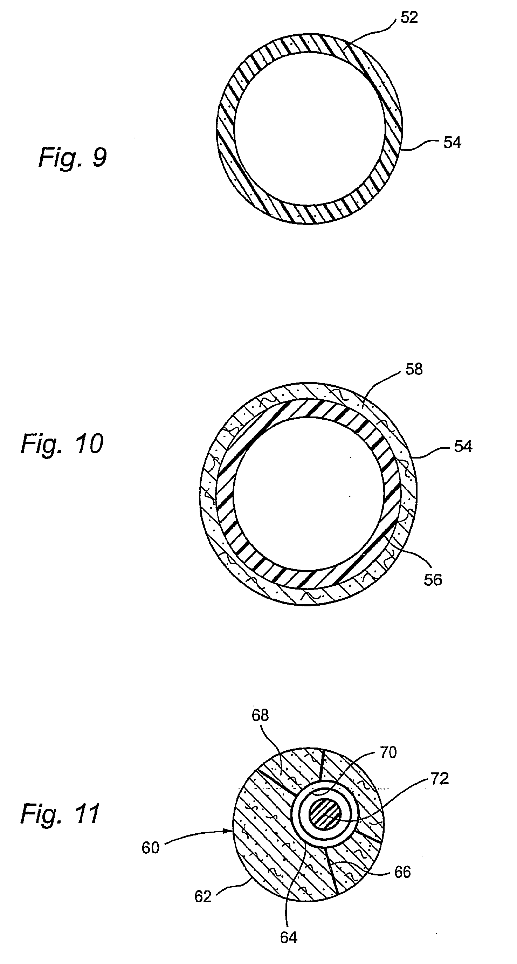

Figure 9 shows a cross-section of a tubular composite structure of an embodiment of the invention, in which the self-supporting base material 52 is made of a polymer material suited for implantation, for example silicone or polyurethane. A property improving coating 54, for example made of Parylene^{™} , Teflon^{™} or metal, is applied on the external surface or on both the external and internal surfaces of the tubular structure

Figure 10 shows the same embodiment as Figure 9 except that the base material comprises a layer 56 of polyurethane surrounded by a layer 58 of silicone (according to the invention).

Figure 11 shows a cross-section of a mechanical constriction device, comprising a double walled tubing 60, an external wall 62 and an internal wall 64 spaced from the external wall 62, of a self-supporting base material of hard silicone. The tubing 60 has partition walls 66 dividing the space between the external and internal walls 62 and 64, respectively, of the tubing 60 into longitudinal cells 68, which are filled with a soft viscoelastic material, for example silicone gel. The internal wall 64 is coated with a friction reducing coating 70, for example made of Teflon™ or the like. A strong band 72 of nylon or the like slides in the tubing 60 on the friction reducing coating 70 to enable adjustment of the constriction device in the same manner as described above in connection with the embodiment according to Figures 1 and 2.

## Claims

1. An implantable constriction device (2) for treating an impotent patient, comprising an elongate composite structure (4) adapted to constrict the exit penile veins or corpus cavernosa of the patient to restrict the penile venous blood flow, wherein said elongate composite structure is composed of a base material (12,32,34,44,50,52,60) making said composite structure self-supporting, **characterized by** a property improving means (14,20,40,46,54,70) for improving at least one physical property of said composite structure other than self-supporting properties, wherein the base material comprises a layer (56) of polyurethane surrounded by a layer (58) of silicone, wherein the property improving means comprises a coating applied on the base material, and wherein the coating applied on the base material is of a material different than the base material.

2. An implantable constriction device according to claim 1, wherein the coating of the property improving means comprises a coating applied on the base material at least along a side of the elongate composite structure that is intended to contact the exit penile veins or corpus cavernosa, the coating having better aggressive body fluid resistant properties than the base material.

3. An implantable constriction device according to claim 2, wherein the base material forms an inflatable tubing.

4. An implantable constriction device according to claim 3, wherein the tubing has an inner surface defining the interior of the tubing, and the coating covers the inner surface.

5. An implantable constriction device according to claim 1, wherein the coating of the property improving means comprises a coating applied on the base material at least along a side of the elongate composite structure that is intended to contact the exit penile veins or corpus cavernosa, the coating having better anti-friction properties than the base material.

6. An implantable constriction device according to claim 2 or 5, wherein the property improving means further comprises a core of a viscoelastic material covered with the self-supporting base material.

7. An implantable constriction device according to claim 5, wherein the base material forms an inflatable tubing.

8. An implantable constriction device according to claim 7, wherein the tubing has an inner surface defining the interior of the tubing, and the coating covers the inner surface.

9. An implantable constriction device according to claim 1, wherein the base material forms two first layers and the coating of the property improving means comprises a coating applied on the first layers, the coating being more fatigue resistant than the first layers,

10. An implantable constriction device according to claim 9, wherein the coating covers the first layers of the base material along a side of the elongate composite structure that is intended to contact the exit penile veins or corpus cavernosa.

11. An implantable constriction device according to claim 9 or 10, wherein the coating comprises a polyurethane layer.

12. An implantable constriction device according to any one of claims 9-11, wherein the property improving means comprises a further coating applied on further coating the coating, the further coating having better aggressive body fluid resistance properties and/or better anti-friction properties than the base material.

13. An implantable constriction device according to claim 12, wherein the further coating is a coating selected from the group consisting of Teflon™, Parylene™, and biocompatible metal coating.

14. An implantable constriction device according to claim 13, wherein the biocompatible metal coating is selected from the group consisting of gold, silver and titanium.

15. An implantable constriction device according to any one of claims 1-9, wherein the first layers of the base material forms an inflatable tubing, and the coating covers the base material within the tubing.

16. An implantable constriction device according to claim 1, wherein the base material forms an inflatable tubing and the coating of the property improving means comprises a liquid impermeable coating applied on the base material.

17. An implantable constriction device according to claim 16, wherein the tubing has an external surface of the base material and an internal surface of the base material defining the interior of the tubing, the coating being applied on the external surface and/or internal surface.

18. An implantable constriction device according to claim 16 or 17, wherein the coating coated on the base material, is a coating being selected from the group consisting of Parylene™ and a biocompatible metal coating.

19. An implantable constriction device according to claim 18, wherein the biocompatible metal coating is selected from the group consisting of gold, silver and titanium.

20. An implantable constriction device according to any one of claims 1-19, wherein hard silicone and polyurethane constitutes the base material.

21. An implantable constriction device according to any one of claims 3, 7 and 16, wherein the base material forms two coaxial tubular layers and the coating of the property improving means comprises a tubular intermediate coating of a soft viscoelastic material located between the coaxial tubular layers.

22. An implantable constriction device according to any one of claims 3, 7 and 16, wherein the base material forms an outer tubular layer and an inner arcuate layer attached to the outer tubular layer, the outer and inner layers defining a curved space extending longitudinally along the tubing, and the coating of the property improving means comprises soft viscoelastic material filling the space.

23. An implantable constriction device according to any one of claims 6, 21 and 22, wherein the viscoelastic material is selected from the group consisting of silicone gel, cellulose gel, and collagen gel.

24. An implantable constriction device according to claim 2, wherein the coating comprises a cell barrier layer applied on the surfaces of the base material to prevent body cells from breaking down the base material.

25. An implantable constriction device according to claim 24, wherein the cell barrier layer is a coating coated on the surfaces of the base material, the coating being selected from the group consisting of Parylene™ and a biocompatible metal coating.

26. An implantable constriction device according to claim 25, wherein the biocompatible metal coating is selected from the group consisting of gold, silver and titanium.

27. An implantable constriction device according to claim 1, wherein the elongate composite structure is non-inflatable.

28. An implantable constriction device according to claim 27, further comprising an adjustment means adapted to mechanically adjust the non-inflatable composite structure to either constrict or release the exit penile veins or corpus cavernosa.

29. An implantable constriction device according to any one of the preceding claims, wherein the elongate composite structure is adapted to externally constrict the stomach or esophagus.

30. An implantable constriction device according to claim 1, wherein the base material forms at least one tubular layer.

31. An implantable constriction device according to claim 1, wherein the coating of the property improving means is applied externally on the tubular layer of the base material.

32. An implantable constriction device according to claim 31, wherein the coating of the property improving means is applied on the tubular layer of the base material at least along a side of the elongate composite structure that is intended to contact the exit penile veins or corpus cavernosa.

33. An implantable constriction device according to claim 1, wherein the coating of the property improving means is applied internally on the tubular layer of the base material.

34. An implantable constriction device according to claim 1, wherein the property improving means comprises a first coating applied externally on the tubular base material and a second coating applied internally on the tubular layer of the base material.

35. An implantable constriction device according to claim 1, wherein the composite structure comprises an external tubular layer of the base material and an internal tubular layer of the base material extending inside of and being spaced from the external tubular layer, whereby the external and internal tubular layers define a space, the coating of the property improving means extending in the space.

## Patentansprüche

1. Implantierbare Einschnüreinrichtung (2) zum Behandeln eines impotenten Patienten, umfassend eine längliche Kompositstruktur (4), ausgebildet zum Einschnüren der Penis-Austrittsvenen oder der Schwellkörper des Patienten, um den venösen Penis-Blutstrom einzuschränken, wobei die längliche Kompositstruktur sich zusammensetzt aus einem Basismaterial (12, 32, 34, 44, 50, 52, 60), welches der Kompositstruktur Selbsttragefähigkeit verleiht, **gekennzeichnet durch** eine Eigenschafts-Verbesserungseinrichtung (14, 20, 40, 46, 54, 70) zum Verbessern mindestens einer physikalischen Eigenschaft der Kompositstruktur außer den selbsttragenden Eigenschaften, wobei das Basismaterial eine Schicht (56) aus Polyurethan aufweist, umgeben von einer Silikonschicht (58), wobei die Eigenschafts-Verbesserungseinrichtung einen auf das Basismaterial aufgebrachten Überzug aufweist, und wobei der auf das Basismaterial aufgebrachte Überzug aus einem von dem Basismaterial verschiedenen Werkstoff besteht.

2. Einrichtung nach Anspruch 1, bei der der Überzug der Eigenschafts-Verbesserungseinrichtung einen Überzug aufweist, der auf das Basismaterial zumindest entlang einer Seite der länglichen Kompositstruktur aufgebracht ist, welche in Kontakt mit den Austrittsvenen des Penis oder den Schwellkörpern treten soll, wobei der Überzug bessere gegenüber aggressiven Körperfluiden resistente Eigenschaften aufweist als das Basismaterial.

3. Einrichtung nach Anspruch 2, bei der das Basismaterial einen aufblähbaren Schlauchkörper bildet.

4. Einrichtung nach Anspruch 3, bei der der Schlauchkörper eine Innenfläche aufweist, die das Innere des Schlauchkörpers bildet, und der Überzug die Innenfläche abdeckt.

5. Einrichtung nach Anspruch 1, bei der der Überzug der Eigenschafts-Verbesserungseinrichtung einen Überzug aufweist, der auf das Basismaterial zumindest entlang einer Seite der länglichen Kompositstruktur aufgebracht ist, die mit den Penis-Austrittsvenen oder den Schwellkörpern in Berührung zu bringen ist, wobei der Überzug besseren Gleiteigenschaften als das Basismaterial besitzt.

6. Einrichtung nach Anspruch 2 oder 5, bei der die Eigenschafts-Verbesserungseinrichtung außerdem einen Kern aus einem viskoelastischen Material aufweist, bedeckt mit dem selbsttragenden Basismaterial.

7. Einrichtung nach Anspruch 5, bei der das Basismaterial ein aufblähbarer Schlauchkörper ist.

8. Einrichtung nach Anspruch 7, bei dem der Schlauchkörper eine Innenfläche aufweist, die das Innere des Schlauchkörpers bildet, wobei der Überzug die Innenfläche bedeckt.

9. Einrichtung nach Anspruch 1, bei der das Basismaterial zwei erste Schichten bildet und der Überzug der Eigenschafts-Verbesserungseinrichtung einen auf die ersten Schichten aufgebrachten Überzug aufweist, wobei der Überzug ermüdungsresistenter ist als die ersten Schichten.

10. Einrichtung nach Anspruch 9, bei der der Überzug die ersten Schichten des Basismaterials entlang einer Seite der länglichen Kompositstruktur abdeckt, die für den Kontakt mit den Penis-Austrittsvenen oder den Schwellkörpern vorgesehen ist.

11. Einrichtung nach Anspruch 9 oder 10, bei der der Überzug eine Polyurethanschicht aufweist.

12. Einrichtung nach einem der Ansprüche 9 bis 11, bei der die Eigenschafts-Verbesserungseinrichtung einen weiteren Überzug aufweist, der auf den Überzug aufgebracht ist, wobei der weitere Überzug bessere gegenüber aggressiven Körperfluiden resistente Eigenschaften und/oder bessere Gleiteigenschaften als das Basismaterial aufweist.

13. Einrichtung nach Anspruch 12, bei der der weitere Überzug ein Überzug ausgewählt aus der Gruppe Teflon^{®}, Parylene^{®} und biokompatiblem Metallüberzug ist.

14. Einrichtung nach Anspruch 13, bei der der biokompatible Metallüberzug ausgewählt ist aus der Gruppe Gold, Silber und Titan.

15. Einrichtung nach einem der Ansprüche 1 bis 9, bei der die ersten Schichten des Basismaterials einen aufblähbaren Schlauchkörper bilden und der Überzug des Basismaterials innerhalb des Schlauchkörpers bedeckt.

16. Einrichtung nach Anspruch 1, bei der das Basismaterial einen aufblähbaren Schlauchkörper aufweist und der Überzug der Eigenschafts-Verbesserungseinrichtung einen flüssigkeitsundurchlässigen Überzug aufweist, der auf das Basismaterial aufgebracht ist.

17. Einrichtung nach Anspruch 16, bei der der Schlauchkörper eine Außenfläche des Basismaterials besitzt und eine Innenfläche des Basismaterials das Innere des Schlauchkörpers definiert, wobei der Überzug auf die Außenfläche und/oder die Innenfläche aufgebracht ist.

18. Einrichtung nach Anspruch 16 oder 17, bei der der auf das Basismaterial aufgebrachte Überzug ein Überzug ist, der ausgewählt ist aus der Gruppe Parylen^{®} und einem biokompatiblen Metallüberzug.

19. Einrichtung nach Anspruch 18, bei der der biokompatible Metallüberzug ausgewählt ist aus der Gruppe Gold, Silber und Titan.

20. Einrichtung nach einem der Ansprüche 1 bis 19, bei der hartes Silikon und Polyurethan das Basismaterial bilden.

21. Einrichtung nach einem der Ansprüche 3, 7 und 16, bei der das Basismaterial 2 koaxiale Schlauchschichten bildet und der Überzug der Eigenschafts-Verbesserungseinrichtung einen schlauchförmigen Zwischenüberzug aus weichem viskoelastischem Material aufweist, da sich zwischen den koaxialen Schlauchschichten befindet.

22. Einrichtung nach einem der Ansprüche 3, 7 und 16, bei der das Basismaterial eine Außen-Schlauchschicht und eine innere gekrümmte Schicht, die an der Außen-Schlauchschicht befestigt ist, bildet, wobei die äußere und die innere Schicht einen gekrümmten Raum bilden, der sich in Längsrichtung entlang dem Schlauchkörper erstreckt, wobei der Überzug der Eigenschafts-Verbesserungseinrichtung weiches viskoelastisches Material aufweist, welches den Raum ausfüllt.

23. Einrichtung nach einem der Ansprüche 6, 21 und 22, bei der das viskoelastische Material ausgewählt ist aus der Gruppe Silikongel, Zellulosegel und Kollagengel.

24. Einrichtung nach Anspruch 2, bei der der Überzug eine Zellen-Barrierenschicht aufweist, die auf die Oberflächen des Basismaterials aufgebracht ist, um zu verhindern, dass Körperzellen das Basismaterial zum Einfallen bringen.

25. Einrichtung nach Anspruch 24, bei dem die Zellen-Barrierenschicht ein auf die Oberflächen des Basismaterials aufgebrachter Überzug ist, wobei der Überzug ausgewählt ist aus der Gruppe Parylen® und einem biokompatiblen Metallüberzug.

26. Einrichtung nach Anspruch 25, bei der der biokompatible Metallüberzug ausgewählt ist aus der Gruppe Gold, Silber und Titan.

27. Einrichtung nach Anspruch 1, bei der die längliche Kompositstruktur nichtaufblähbar ist.

28. Einrichtung nach Anspruch 27, weiterhin umfassend eine Einstelleinrichtung, ausgebildet zum mechanischen Justieren der nicht-aufblähbaren Kompositstruktur, um die Penis-Austrittsvenen oder die Schwellkörper entweder einzuschnüren oder freizugeben.

29. Einrichtung nach einem der vorhergehenden Ansprüche, bei der die längliche Kompositstruktur dazu ausgebildet ist, den Magen oder die Speiseröhre von außen einzuschnüren.

30. Einrichtung nach Anspruch 1, bei dem das Basismaterial mindestens eine Schlauchschicht bildet.

31. Einrichtung nach Anspruch 1, bei der der Überzug der Eigenschafts-Verbesserungseinrichtung von außen auf die Schlauchschicht des Basismaterials aufgebracht ist.

32. Einrichtung nach Anspruch 31, bei der die Eigenschafts-Verbesserungseinrichtung auf die Schlauchschicht des Basismaterials mindestens entlang einer Seite der länglichen Kompositstruktur aufgebracht ist, die für den Kontakt mit den Penis-Austrittsvenen oder den Schwellkörpern vorgesehen ist.

33. Einrichtung nach Anspruch 1, bei der der Überzug der Eigenschafts-Verbesserungseinrichtung innen auf die Schlauchschicht des Basismaterials aufgebracht ist.

34. Einrichtung nach Anspruch 1, bei der die Eigenschafts-Verbesserungseinrichtung einen ersten Überzug aufweist, der außen auf das schlauchförmige Basismaterial aufgebracht ist, und einen zweiten Überzug aufweist, der innen auf die Schlauchschicht des Basismaterials aufgebracht ist.

35. Einrichtung nach Anspruch 1, bei der die Kompositstruktur eine äußere Schlauchschicht des Basismaterials und eine innere Schlauchschicht des Basismaterials aufweist, wobei letztere sich auf der Innenseite erstreckt und von der äußeren Schlauchschicht beabstandet ist, wodurch die äußere und die innere Schlauchschichten einen Raum definieren, und der Überzug der Eigenschafts-Verbesserungseinrichtung sich innerhalb des Raumes erstreckt.

## Revendications

1. Dispositif de constriction implantable (2) pour traiter un patient impuissant, comprenant une structure composite allongée (4) qui est adaptée pour serrer les veines péniennes de sortie ou le corps caverneux du patient afin de limiter le flux sanguin dans les veines péniennes dans lequel, ladite structure composite allongée est constituée d'un matériau de base (12, 32, 34, 44, 50, 52, 60) rendant autoporteuse ladite structure composite, **caractérisé par** un moyen d'amélioration de propriétés (14, 20, 40, 46, 54, 70) pour améliorer au moins une propriété physique de ladite structure composite autre que des propriétés de structure autoporteuse dans lequel, le matériau de base comprend une couche (56) de polyuréthane entourée d'une couche (58) de silicone, dans lequel le moyen d'amélioration de propriétés comprend un revêtement appliqué sur le matériau de base, et dans lequel le revêtement appliqué sur le matériau de base est en un matériau différent du matériau de base.

2. Dispositif de constriction implantable selon la revendication 1, dans lequel le revêtement du moyen d'amélioration de propriétés comprend un revêtement appliqué sur le matériau de base au moins le long d'un côté de la structure composite allongée qui est destiné à venir en contact avec les veines péniennes de sortie ou le corps caverneux, le revêtement présentant de meilleures propriétés de résistance aux fluides corporels agressifs que le matériau de base.

3. Dispositif de constriction implantable selon la revendication 2, dans lequel le matériau de base forme un conduit gonflable.

4. Dispositif de constriction implantable selon la revendication 3, dans lequel le conduit présente une surface intérieure définissant le revêtement intérieur du conduit, et le revêtement recouvre la surface intérieure.

5. Dispositif de constriction implantable selon la revendication 1, dans lequel le revêtement du moyen d'amélioration de propriétés comprend un revêtement appliqué sur le matériau de base au moins le long d'un côté de la structure composite allongée qui est destiné à venir en contact avec les veines péniennes de sortie ou le corps caverneux, le revêtement présentant de meilleures propriétés d'antifriction que le matériau de base.

6. Dispositif de constriction implantable selon la revendication 2 ou 5, dans lequel le moyen d'amélioration de propriétés comprend en outre un noyau en matériau viscoélastique recouvert du matériau de base autoporteur.

7. Dispositif de constriction implantable selon la revendication 5, dans lequel le matériau de base forme un conduit gonflable.

8. Dispositif de constriction implantable selon la revendication 7, dans lequel le conduit présente une surface intérieure définissant l'intérieur du conduit, et le revêtement recouvre la surface intérieure.

9. Dispositif de constriction implantable selon la revendication 1, dans lequel le matériau de base forme deux premières couches et le revêtement du moyen d'amélioration de propriétés comprend un revêtement appliqué sur la première couche, le revêtement étant plus résistant à la fatigue que les premières couches.

10. Dispositif de constriction implantable selon la revendication 9, dans lequel le revêtement recouvre les premières couches de matériau de base le long d'un côté de la structure composite allongée qui est destiné à venir en contact avec les veines péniennes de sortie ou le corps caverneux.

11. Dispositif de constriction implantable selon la revendication 9 ou 10, dans lequel le revêtement comprend une couche de polyuréthane.

12. Dispositif de constriction implantable selon l'une quelconque des revendications 9 à 11, dans lequel le moyen d'amélioration de propriétés comprend un revêtement supplémentaire appliqué sur le revêtement, le revêtement supplémentaire présentant de meilleures propriétés de résistance aux fluides corporels agressifs et/ou de meilleures propriétés d'antifriction que le matériau de base.

13. Dispositif de constriction implantable selon la revendication 12, dans lequel le revêtement supplémentaire est un revêtement sélectionné dans le groupe constitué du Téflon™, du Parylène™, et d'un revêtement métallique biocompatible.

14. Dispositif de constriction implantable selon la revendication 13, dans lequel le revêtement métallique biocompatible est sélectionné dans le groupe constitué de l'or, de l'argent et du titane.

15. Dispositif de constriction implantable selon l'une quelconque des revendications 1 à 9, dans lequel les premières couches de matériau de base forment un conduit gonflable, et le revêtement recouvre le matériau de base à l'intérieur du conduit.

16. Dispositif de constriction implantable selon la revendication 1, dans lequel le matériau de base forme un conduit expansible et le revêtement du moyen d'amélioration de propriétés comprend un revêtement imperméable aux liquides appliqué sur le matériau de base.

17. Dispositif de constriction implantable selon la revendication 16, dans lequel le conduit présente une surface extérieure de matériau de base et une surface intérieure de matériau de base définissant l'intérieur du conduit, le revêtement étant appliqué sur la surface extérieure et/ou la surface intérieure.

18. Dispositif de constriction implantable selon la revendication 16 ou 17, dans lequel le revêtement recouvrant le matériau de base est un revêtement qui est sélectionné dans le groupe constitué du Parylène^{™} et d'un revêtement métallique biocompatible.

19. Dispositif de constriction implantable selon la revendication 18, dans lequel le revêtement métallique biocompatible est sélectionné dans le groupe constitué de l'or, l'argent et le titane.

20. Dispositif de constriction implantable selon l'une quelconque des revendications 1 à 19, dans lequel le revêtement de silicone dur et le polyuréthane constituent le matériau de base.

21. Dispositif de constriction implantable selon l'une quelconque des revendications 3, 7 et 16, dans lequel le matériau de base forme deux couches tubulaires coaxiales et le revêtement du moyen d'amélioration de propriétés comprend un revêtement intermédiaire tubulaire de matériau viscoélastique souple situé entre les couches tubulaires coaxiales.

22. Dispositif de constriction implantable selon l'une quelconque des revendications 3, 7 et 16, dans lequel le matériau de base forme une couche tubulaire extérieure et une couche incurvée intérieure fixée à la couche tubulaire extérieure, les couches extérieure et intérieure définissant un espace courbe s'étendant longitudinalement le long du conduit, et le revêtement du moyen d'amélioration de propriétés comprend un matériau viscoélastique souple remplissant l'espace.

23. Dispositif de constriction implantable selon l'une quelconque des revendications 6, 21 et 22, dans lequel le matériau viscoélastique est sélectionné dans le groupe constitué de gel de silicone, de gel de cellulose, et de gel de collagène.

24. Dispositif de constriction implantable selon la revendication 2, dans lequel le revêtement comprend une couche de barrière cellulaire appliquée sur les surfaces du matériau de base pour empêcher des cellules corporelles de décomposer le matériau de base.

25. Dispositif de constriction implantable selon la revendication 24, dans lequel la couche de barrière cellulaire est un revêtement recouvrant les surfaces du matériau de base, le revêtement étant sélectionné dans le groupe constitué du Parylène™ et d'un revêtement métallique biocompatible.

26. Dispositif de constriction implantable selon la revendication 25, dans lequel le revêtement métallique biocompatible est sélectionné dans le groupe constitué de l'or, l'argent et le titane.

27. Dispositif de constriction implantable selon la revendication 1, dans lequel la structure composite allongée est non expansible.

28. Dispositif de constriction implantable selon la revendication 27, comprenant en outre un moyen d'ajustement adapté pour ajuster mécaniquement la structure composite non expansible soit pour serrer soit pour libérer les veines péniennes de sortie ou le corps caverneux.

29. Dispositif de constriction implantable selon l'une quelconque des revendications précédentes, dans lequel la structure composite allongée est adaptée pour serrer extérieurement l'estomac ou l'oesophage.

30. Dispositif de constriction implantable selon la revendication 1, dans lequel le matériau de base forme au moins une couche tubulaire.

31. Dispositif de constriction implantable selon la revendication 1, dans lequel le revêtement du moyen d'amélioration de propriétés est appliqué extérieurement sur la couche tubulaire du matériau de base.

32. Dispositif de constriction implantable selon la revendication 31, dans lequel le revêtement du moyen d'amélioration de propriétés est appliqué sur la couche tubulaire du matériau de base au moins le long d'un côté de la structure composite allongée qui est destiné à venir en contact avec les veines péniennes de sortie ou le corps caverneux.

33. Dispositif de constriction implantable selon la revendication 1, dans lequel le revêtement du moyen d'amélioration de propriétés est appliqué intérieurement sur la couche tubulaire du matériau de base.

34. Dispositif de constriction implantable selon la revendication 1, dans lequel le moyen d'amélioration de propriétés comprend un premier revêtement appliqué extérieurement sur le matériau de base tubulaire et un second revêtement appliqué intérieurement sur la couche tubulaire du matériau de base.

35. Dispositif de constriction implantable selon la revendication 1, dans lequel la structure composite comprend une couche tubulaire extérieure de matériau de base et une couche tubulaire intérieure de matériau de base s'étendant à l'intérieur et à distance de la couche tubulaire extérieure, les couches tubulaires extérieure et intérieure définissant un espace, le revêtement du moyen d'amélioration de propriétés s'étendant dans l'espace.
